# EUROPÄISCHE PATENTANMELDUNG

(11) **EP 2 266 570 A1**
(43) Veröffentlichungstag der Anmeldung: **29.12.2010**
(21) Anmeldenummer: 10178088.0
(22) Anmeldetag: 11.01.2000
(51) Int. Cl.: A61K 31/505, A61P 25/18, A61P 25/00

(54) **Verwendung von 5-ht5-liganden zur behandlung neurodegenerativer und neuropsychiatrischer störungen**

(30) Priorität: 11.01.1999 DE 19900673
(62) Teilanmeldung aus: 00904894.3
(71) Anmelder: Abbott GmbH & Co. KG, 65205 Wiesbaden (DE)
(72) Erfinder: Garcia-Ladona, , Francisco Javier, 76870, Kandel (DE); Szabo, Lazlo, 69221, Dossenheim (DE); Steiner, Gerd, 67281, Kirchheim (DE); Hofmann, Hans-Peter, 67117, Limburgerhof (DE)
(74) Vertreter: Reitstötter - Kinzebach

(57) **Zusammenfassung**

Die vorliegende Erfindung betrifft die Verwendung von Bindungspartnern für 5-HT5-Rezeptoren zur Behandlung neuropsychiatrischer Störungen wie Schizophrenie und Psychosen.

## Beschreibung

Die vorliegende Erfindung betrifft die Verwendung von Bindungspartnern für 5-HT5-Rezeptoren zur Behandlung neuropathologischer Störungen sowie damit zusammenhängender Anzeichen, Symptome und Fehlfunktionen und Verfahren zur Identifizierung und Charakterisierung solcher Bindungspartner.

Wenigstens sieben verschiedene Rezeptorklassen vermitteln die mannigfaltigen physiologischen Aktivitäten, die einer Beteiligung des Neurotransmitters Serotonin (5-Hydroxytryptamin, kurz 5-HT) zugeschrieben werden. Sie werden entsprechend einer international anerkannten Klassifikation mit 5-HT1, 5-HT2, 5-HT3, 5-HT4, 5-HT5, 5-HT6 und 5-HT7 bezeichnet. Die meisten dieser Klassen umfassen darüber hinaus weitere unterscheidbare Rezeptortypen. So gehören zur 5-HTI-Klasse Rezeptoren, welche sich in wenigstens fünf Unterklassen einteilen lassen, die mit 5-HT1A, 5-HT1B, 5-HT1C, 5-HT1D und 5-HT1E bezeichnet werden (Boess F.G. und Martin I.L., Neuropharmacology 33:275-317 (1994)).

Die 5-HT5-Klasse wurde erstmals beschrieben von Plassat et al., The EMBO Journal Bd. 11 Nr. 13, S. 4779-4786 (1992). Man unterscheidet 5-HT5A- und 5-HT5B-Rezeptoren (Erlander et al., Proc. Natl. Acad. Sci. USA 90:3452-3456 (1993)). Es bestehen nur geringe Sequenzhomologien zwischen 5-HT5 und anderen 5-HT-Rezeptoren. Das pharmakologische Profil dieser Rezeptoren unterscheidet sich deutlich. Mit molekularbiologischen Techniken gelang die Lokalisierung von 5-HT5-Rezeptoren im Riechkolben, im Hippocampus, im Cortex, in den Zerebralventrikeln, im Corpus Callosum und im Kleinhirn. Mittels immunhistochemischer Methoden konnte gezeigt werden, daß 5-HT5-Rezeptoren vornehmlich auf Astrocyten exprimiert werden (Carson et al., GLIA 17:317-326 (1996)). Astrocyten liegen direkt an der Basalmembran von Gehirnkapillaren der Bluthirnschranke an. Eine anormale Astrocyten-Endothelium-Struktur geht mit einem Verlust der Bluthirnschranke einher. Die genaue Bedeutung der Astrocyten ist unklar. Sie scheinen Transportaufgaben und konnektive Funktionen wahrzunehmen. Reaktive Astrocyten wurden in Zusammenhang mit reaktiver Gliosis bei einer Reihe von pathologischen Gehirnveränderungen und neuropsychiatrischen Erkrankungen beobachtet. Infolge von Gehirnverletzungen verändern sie ihre Morphologie. Das Proteinexpressionsmuster ändert sich und Wachstumsfaktoren werden produziert. In vitro-Untersuchungen an kultivierten Astrocyten haben 5-HT5-Rezeptor-vermittelte Antworten erkennen lassen. So wird einerseits vermutet, daß sie an Erholungsprozessen des Gehirns nach Störungen beteiligt sind, andererseits ist aber auch nicht auszuschließen, daß sie zur Schadensentstehung oder sogar zu einer Schadensvermehrung beitragen.

ZNS-Erkrankungen betreffen heutzutage große Bevölkerungsteile. Insbesondere aufgrund der Zunahme älterer Menschen steigen die Patientenzahlen ständig. Neuropathologische Zustände wie cerebrale Ischämie, Schlaganfall, Epilepsie und Anfälle im allgemeinen, chronische Schizophrenie, andere psychotischen Erkrankungen, Demenz, insbesondere Alzheimer Demenz, demyelinisierende Erkrankungen, insbesondere Multiple Sklerose, und Gehirntumore führen zu Schädigungen des Gehirns und den damit verbundenen neuronalen Defiziten.

Therapeutische Behandlungen der geschilderten neurodegenerativen und neuropsychiatrischen Störungen richteten sich bislang auf verschiedene Membranrezeptoren mit dem Ziel, Defizite in Neurotransmissionsvorgängen zu kompensieren. Zwar konnten neuroprotektive Wirkungen mit serotoninergen Verbindungen in Tiermodellen für neuropathologische Zustände, wie Ischämie, Hirnschlag und Excitotoxizität, erzielt werden. Teilweise konnten auch günstige Wirkungen auf Gemütsstörungen, wie Depression oder Angstzustände, beobachtet werden. Zu nennen sind hier beispielsweise 5-HT1A-Agonisten, wie Buspiron, oder die als selektiver 5-HT1A-Rezeptor-Ligand charakterisierte Verbindung 8-Hydroxy-2-(di-n-propylamino)tetralin (8-OH-DPAT). Diese Wirkstoffe mindern neurologische Defizite allerdings nur bedingt. Eine effektive Therapie gibt es derzeit noch nicht.

Aufgabe der vorliegenden Erfindung ist es daher, die Behandlung neuropathologischer Störungen mit ausreichender Wirksamkeit und geringen Nebenwirkungen zu ermöglichen.

Überraschenderweise wurde nun gefunden, daß durch gezielte Verwendung von Substanzen mit Bindungsaffinitäten für 5-HT5-Rezeptoren eine Behandlung obiger Krankheitszustände sowie damit zusammenhängender Anzeichen, Symptome und Fehlfunktionen ermöglicht wird.

Ein Gegenstand der vorliegenden Erfindung ist daher die Verwendung von Bindungspartnern für 5-HT5-Rezeptoren zur Herstellung eines Mittels zur Behandlung neuropathologischer Störungen sowie damit zusammenhängender Anzeichen, Symptome und Fehlfunktionen.

Unter neuropathologischen Störungen versteht man erfindungsgemäß Störungen, die von neurologischen Defiziten begleitet sind, d. h. einen durch neurologische Ausfallerscheinungen gekennzeichneten Zustand. Der Begriff "Störung" im erfindungsgemäßen Sinne bezeichnet Anomalien, die in der Regel als krankhafte Zustände angesehen werden und sich in Form bestimmter Anzeichen, Symptome und/oder Fehlfunktionen zu erkennen geben können. Die erfindungsgemäße Behandlung kann auf einzelne Störungen sprich Anomalien bzw. krankhafte Zustände gerichtet sein, es können aber auch mehrere gegebenenfalls ursächlich miteinander verbundene Anomalien zu Mustern, d.h. Syndromen, zusammengefaßt sein, die erfindungsgemäß behandelt werden können.

Dieser Zustand kann vorübergehend, fortschreitend oder persistierend bestehen.

Erfindungsgemäß bevorzugt ist die Behandlung neurodegenerativer und/oder neuropsychiatrischer Störungen. Diese Störungen treten insbesondere bei neuropathologischen, in der Regel Gehirnschädigungen verursachenden Krankheitsbildern auf, bei-spielsweise cerebraler Ischämie, Schlaganfall, Epilepsie und Anfällen im allgemeinen, chronischer Schizophrenie, anderen psychotischen Erkrankungen, Demenz, insbesondere Alzheimer Demenz, demyelinisierenden Erkrankungen, insbesondere Multipler Sklerose, und Gehirntumoren. Gegenstand der Erfindung ist insbesondere auch die Verwendung von 5-HT5-Bindungspartnern für die Behandlung solcher Formen oben aufgeführter Erkrankungen, an deren Entstehung und/oder Verlauf 5-HT5-Rezeptoren beteiligt sind, d.h. Erkrankungen, die durch eine 5-HT5-Rezeptoraktivität moduliert werden.

Gemäß eines weiteren Aspekts der vorliegenden Erfindung werden neuropathologische Störungen behandelt, die mit einer glialen Reaktion einhergehen. Die erfindungsgemäße Verwendung betrifft insbesondere die Modulation einer glialen Reaktion.

Eine vorteilhafte Wirkung der Bindungspartner zeigt sich bei der präventiven oder akuten Behandlung neurologischer Defizite, die an Patienten beobachtet werden, die unter psychiatrischen Erkrankungen leiden, wie Epilepsie, Psychose, z.B. Psychosen vom akuten exogenen Reaktionstyp oder Begleit-Psychosen organischer bzw. exogener Ursache, z.B. nach Trauma, vor allem Hirnläsionen und diffusen Hirnschädigungen, bei Stoffwechselstörungen, Infektionen, und Endokrinopathien; endogene Psychosen, wie Schizophrenie sowie schizotype und wahnhafte Störungen; affektive Störungen, wie Depression, Manie bzw. manisch-depressive Zustände; sowie Mischformen der zuvor geschilderten Psychosen; seniler Demenz und seniler Demenz vom Alzheimer Typ, sowie bei der Behandlung oder Prävention von Demyelinisationsvorgängen.

Wirksam sind die erfindungsgemäßen Bindungspartner insbesondere im Hinblick auf die Behandlung ischämischer Schäden, z.B. infolge von Hirn- und Rückenmarkstrauma sowie Gefäßverschluß oder Herzversagen.

Zu nennen ist hier vor allem der Schlaganfall (Synonym: Apoplexia cerebri, cerebraler oder apoplektischer Insult, Gehirnschlag). Erfindungsgemäß behandelbar sind transitorisch-ischämische Attakken, reversible ischämische neurologische Defizite, prolongierte reversible ischämische neurologische Defizite, partiell reversible ischämische neurologische Symptomatiken und auch persistierende komplette Hirninfarkte. Besonders vorteilhaft ist erfindungsgemäß die Behandlung akuter Formen.

Den erfindungsgemäß bevorzugt behandelten Formen neuropathologischer Störungen liegen eine oder mehrere der nachfolgend aufgezählten Veränderungen von Nervengeweben zugrunde: Degeneration oder Absterben von Neuronen, insbesondere der Ganglienzellen, z.B. Tigrolyse, Kernmembranunschärfe, Zellschrumpfung, Zytoplasmavakuolisierung und -inkrustation, Parenchymnekrosen des Gehirns, Hirnödeme, durch Sauerstoffmangel verursachte Veränderungen von Neuronen, Atrophie, morphologische Veränderungen, wie Demyelinisierungen, insbesondere ein Markscheidenzerfall, perivaskuläre Infiltrate, gliöse Proliferation und/oder Glianarben; Degeneration der Substantia nigra.

Die erfindungsgemäß zu behandelnde Indikation ist häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten.

Durch die erfindungsgemäße Behandlung neuropathologischer Störungen bzw. den ihr zugrundeliegenden Zuständen lassen sich eine Reihe weiterer Anzeichen, Symptome und/oder Fehlfunktionen behandeln, die mit den neuropathologischen Störungen zusammenhängen, d.h. insbesondere die oben beschriebenen Erkrankungszustände begleiten. Hierzu gehören beispielsweise Schocklunge; Hirnnervenausfälle, z.B. retrobulbäre Neuritis, Augenmuskellähmungen, skandierende Sprache, spastische Lähmungen, Kleinhirnsymptome, Sensibilitäts-, Blasen- und Mastdarmstörungen, Euphorie, Demenz; Hypo- und Akinese, fehlende Mitbewegung, kleinschrittiger Gang, Beugehaltung von Rumpf und Gliedern, Pro-, Retro- und Lateropulsion, Tremor, Mimikarmut, monotone Sprache, Depressionen, Apathie, labile oder starre Affektivität, erschwerte Spontaneität und Entschlußkraft, velangsamtes Denken, verarmte Assoziationsfähigkeit; Muskelatrophie.

Eine Behandlung im erfindungsgemäßen Sinne umfasst nicht nur die Behandlung akuter oder chronischer Anzeichen, Symptome und/oder Fehlfunktionen sondern auch eine vorbeugende Behandlung (Prophylaxe) insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Der Begriff "Bindungspartner für 5-HT5-Rezeptoren" beschreibt Substanzen, welche an 5-HT5-Rezeptoren binden und daher auch als 5-HT5-Rezeptorliganden bezeichnet werden können.

Unter Bindung versteht man jede molekulare Wechselwirkung zwischen dem Bindungspartner und dem Rezeptor, insbesondere unter physiologischen Bedingungen. Dies sind in der Regel klassische Wechselwirkungen, zu denen elektrostatische Anziehung, Wasserstoffbrücken-Bindung, hydrophobe Bindungen, van-der-Waals-Kräfte oder metallkomplexartige koordinative Bindungen gehören. Zusätzlich zu den vorstehend genannten, reversiblen molekularen Wechselwirkungen können auch irreversible Wechselwirkungen zwischen Bindungspartner und Rezeptor in Betracht kommen, wie z.B. kovalente Bindungen.

Gemäß einer Ausführungsform hemmen erfindungsgemäß verwendbare Bindungspartner die Bindung von Vergleichsbindungs-partnern, wie 5-HT (5-Hydroxytryptamin) oder 5-CT (5-Carboxamidotryptamin), an 5-HT5-Rezeptoren kompetitiv.

Unter kompetitiver Hemmung versteht man, daß erfindungsgemäß verwendbare Bindungspartner mit einem Vergleichsbindungspartner, im vorliegenden Fall z.B. 5-HT oder 5-CT, um die Bindung an den Rezeptor konkurrieren, d.h. die Bindung des einen behindert die Bindung des anderen.

Gemäß einer weiteren Ausführungsform hemmen erfindungsgemäß verwendbare Bindungspartner die Bindung von Vergleichsbindungs-partnern, wie 5-HT (5-Hydroxytryptamin) oder 5-CT (5-Carboxamidotryptamin), an 5-HT5-Rezeptoren nicht-kompetitiv.

Unter nicht-kompetitiver Hemmung versteht man, daß erfindungsgemäß verwendbare Bindungspartner über ihre Bindung an den Rezeptor die Bindung eines Vergleichsbindungspartner, im vorliegenden Fall z.B. 5-HT oder 5-CT, modulieren, insbesondere dessen Bindungsaffinität verringern.

Zumindest für den Fall der kompetitiven Hemmung, also der reversiblen Bindung gilt der Grundsatz, daß die Verdrängung eines Bindungspartners durch einen anderen mit abnehmender Bindungsaffinität des einen bzw. zunehmender Bindungsaffinität des anderen im Hinblick auf den Rezeptor zunimmt. Zweckmäßigerweise besitzen daher erfindungsgemäß verwendbare Bindungspartner eine hohe Bindungsaffinität für 5-HT5-Rezeptoren. Eine derartige Bindungsaffinität gestattet einerseits eine wirksame Verdrängung natürlich vorkommender Bindungspartner für 5-HT5-Rezeptoren, wie beispielsweise Serotonin (5-Hydroxytryptamin, 5-HT) selbst, wobei die erforderliche Konzentration an erfindungsgemäß verwendbarem Bindungspartner zur Bindung einer bestimmten Menge dieses Bindungspartners an 5-HT5-Rezeptoren mit zunehmender Bindungsaffinität abnimmt. Im Hinblick auf die medizinische Anwendung werden daher Bindungspartner bevorzugt, deren Bindungsaffinität so groß ist, daß diese als Wirkstoff im Rahmen einer wirksamen medizinischen Behandlung in vertretbaren Mengen verabreicht werden können. Erfindungsgemäß verwendbare Bindungspartner werden daher vorzugsweise in Tagesdosen von etwa 0,01 bis 100 mg/kg Körpergewicht und insbesondere von etwa 0,1 bis 15 mg/kg Körpergewicht bei parenteraler Gabe und 1 bis 30 mg/kg Körpergewicht bei oraler Gabe verabreicht.

Eine Möglichkeit, die Bindungsaffinität auszudrücken, bieten die oben angesprochenen Kompetitionsexperimente, mit denen man in vitro diejenige Konzentration an erfindungsgemäß verwendbarem Bindungspartner ermittelt, die einen anderen Vergleichsbindungspartner zu 50% von der Rezeptorbindungsstelle verdrängt (IC₅₀-Werte). So läßt sich auch die kompetitive Hemmung der Bindung von 5-CT an 5-HT5-Rezeptoren dahingehend auswerten, daß erfindungsgemäß bevorzugt verwendbare Bindungspartner halbmaximale Hemmkonstanten IC₅₀ von weniger als 10⁻⁵ M, vorzugweise von weniger als 10⁻⁶ M und insbesondere von weniger als 10⁻⁷ M aufweisen.

Die Bindungsaffinität erfindungsgemäß brauchbarer Bindungspartner kann auch über die Hemmkonstante Kᵢ ausgedrückt werden, die man im allgemeinen ebenfalls mit Kompetitionsexperimenten in vitro bestimmt. Für die Bindung an 5-HT5-Rezeptoren weisen erfindungsgemäß verwendbare Bindungspartner vorzugsweise Ki-Werte von weniger als 10⁻⁶ M, vorteilhafterweise von weniger als 10⁻⁷ M und insbesondere bevorzugt von weniger als 10⁻⁸ M auf. Ki-Werte erfindungsgemäß brauchbarer Verbindungen liegen z.B. im Bereich von 1·10⁻⁷ M bis 7·10⁻⁷ M oder im Bereich von 1·10⁻⁸ M bis 1·10⁻⁷ M.

Brauchbare Bindungspartner können mit einer geringeren, einer im wesentlichen gleichen, oder einer höheren Affinität an 5-HT5 binden als an einen bestimmten von 5-HT5 verschiedenen Rezeptor.

So gehören zu Bindungspartnern für 5-HT5-Rezeptoren im Hinblick auf die erfindungsgemäße Verwendung insbesondere diejenigen, deren Bindungsaffinität zu 5-HT5-Rezeptoren verglichen mit der Affinität zu 5-HT1-Rezeptoren, insbesondere 5-HT1A, 5-HT1B und/oder 5-HT1D, so hoch ist, daß sie für die erfindungsgemäße Verwendung in vorteilhafter Weise geeignet sind. Dies setzt nicht notwendigerweise eine vergleichsweise selektivere Bindung an 5-HT5-Rezptoren voraus, wenngleich selektive Bindungspartner für 5-HT5-Rezptoren eine besondere Ausführungsform der vorliegenden Erfindung sind.

Beispielsweise kann man Bindungspartner verwenden, die hochaffin sind sowohl zu 5-HT5 als auch zu 5-HT1-Rezeptoren, insbesondere zu 5-HT1A, 5-HT1B und/oder 5-HT1D. Hochaffin bedeutet in diesem Zusammenhang Ki-Werte in der Regel im Bereich von 1·10⁻⁹ M bis 1·10⁻⁶ M. Gemäß einer besonderen Ausführungsform besitzen brauchbare Bindungspartner im hochaffinen Bereich zu 5-HT-Rezeptoren ein Bindungsprofil, das durch eine Bindungsaffinität zu 5-HT5 gekennzeichnet ist, die im Vergleich zu anderen Bindungsaffinitäten dieses Bereichs im wesentlichen gleich oder nur wenig geringer ist. Faktoren von 10 oder weniger können von Vorteil sein.

Erfindungsmemäß verwendbare selektive Bindungspartner besitzen Bindungsaffinitäten für 5-HT5-Rezeptoren, die größer sind als für einen oder mehrere von 5-HT5 verschiedene 5-HT-Rezeptoren, also insbesondere den obengenannten 5-HT-Rezeptorklassen 5-HT1, 5-HT2, 5-HT3, 5-HT4, 5-HT6 und 5-HT7 zuzuordnenden Rezeptoren. Ist die Bindungsaffinität für 5-HT5-Rezeptoren eines Bindungspartners größer als die eines von 5-HT5 verschiedenen 5-HT-Rezeptors, so spricht man von einer in Bezug auf den von 5-HT5 verschiedenen 5-HT-Rezeptor selektiven Bindung dieser Bindungspartner an 5-HT5-Rezeptoren. Besondere Bindungspartner sind diejenigen, deren Bindungsaffinität für 5-HT5-Rezeptoren größer ist als für wenigstens einen und insbesondere sämtliche 5-HT1-Rezeptoren, insbesondere für 5-HT1A-, 5-HT1D- und/oder 5-HT1B-Rezeptoren. Bindungspartner, deren Bindungsaffinität für 5-HT5-Rezeptoren größer ist als für sämtliche von 5-HT5 verschiedene 5-HT-Rezeptoren, stellen eine weitere besondere Klasse erfindungsgemäßer Bindungspartner dar.

Unter Selektivität versteht man die Eigenschaft eines Bindungspartners, vorzugsweise an 5-HT5-Rezeptoren zu binden.

Für die vorstehend geschilderte Selektivität ist maßgebend, daß sich die Bindungsaffinitäten für 5-HT5-Rezeptoren einerseits und für einen oder mehrere von 5-HT5 verschiedene 5-HT-Rezeptoren andererseits hinreichend unterscheiden. Bevorzugt sind Affinitätsunterschiede, wonach Bindungsaffinitäts-Verhältnisse von wenigstens 2, vorteilhafter von wenigstens 5, besonders vorteilhaft von wenigstens 10, vorzugsweise von wenigstens 20, besonders bevorzugt von wenigstens 50 und insbesondere von wenigstens 100 vorliegen.

Gemäß einer weiteren Ausführungsform binden erfindungsgemäß brauchbare Bindungspartner in Bezug auf einen oder mehrere von 5-HT5 verschiedene 5-HT-Rezeptoren selektiv an 5-HT5-Rezeptoren mit den oben beschriebenen vorteilhaften Bindungsaffinitäten.

Gemäß einer weiteren Ausführungsform binden erfindungsgemäß brauchbare Bindungspartner in Bezug auf alle von 5-HT5 verschiedenen 5-HT-Rezeptoren selektiv an 5-HT5-Rezeptoren mit den oben beschriebenen vorteilhaften Bindungsaffinitäten.

Besonders vorteilhaft sind Bindungspartner, die mit den vorstehend beschriebenen Affinitäten und Selektivitäten an 5-HT5-Rezeptoren binden, die von Gliazellen und insbesondere von Astrocyten exprimiert werden.

Erfindungsgemäß ist die humane Rezeptorvariante bevorzugtes Target für die erfindungsgemäß eingesetzten Bindungspartner.

Die Bindung erfindungsgemäßer Bindungspartner an 5-HT5-Rezeptoren ist an eine Effektorfunktion gekoppelt. Bindungspartner können agonistisch oder antagonistisch sowie teilagonistisch und/oder teilantagonistisch wirken.

Als Agonisten werden erfindungsgemäß Verbindungen bezeichnet, die ganz oder teilweise die Aktivität von 5-HT an 5-HT5-Rezeptoren nachahmen.

Als Antagonisten werden erfindungsgemäß Verbindungen bezeichnet, welche die agonistische Aktivität von 5-HT an 5-HT5-Rezeptoren blockieren können.

Gemäß einer besonderen Ausführungsform der vorliegenden Erfindung werden Bindungspartner eingesetzt, deren Bindung zumindest an 5-HT5-Rezeptoren h5-HT5-transfizierter CHO-Zellen eine Veränderung der Agonist-induzierten Stimulierung der GTP-Bindung an membrangebundene G-Proteine, eine Veränderung intrazellulärer Calcium-Spiegel, eine Veränderung der Agonist-induzierten Induktion der Phospholipase C-Aktivität und/oder eine Veränderung der cAMP-Produktion bewirkt. Was die Veränderung intrazellulärer Calcium-Spiegel angeht, so stellt die Verwendung von Bindungspartnern, die eine Erhöhung intrazellulärer Calcium-Spiegel bewirken, eine besondere Ausführungsform der Erfindung dar.

Zu dieser Ausführungsform gehören auch Bindungspartner, die in bekannten Tiermodellen für neurodegenarative und neuropsychiatrische Vorgänge wirksam sind.

Bevorzugt sind Bindungspartner, die auch in Bezug auf ihre Effektorfunktion im oben beschriebenen Sinn selektiv für 5-HT5-Rezeptoren sind.

Erfindungsgemäß brauchbare Verbindungen sind beispielweise in der DE 197 24 979.5 beschrieben. Hierbei handelt es sich um 3-substituierte 3,4,5,6,7,8-Hexahydro-pyrido[3',4':4,5]thieno[2,3-d]pyrimidin-Derivate der Formel I 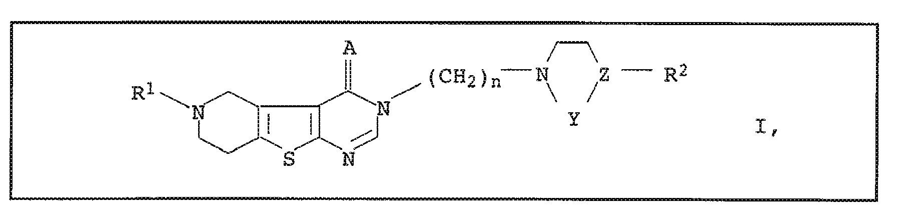 worin
R¹ ein Wasserstoffatom, eine C₁-C₄-Alkylgruppe, eine Acetylgruppe, ein Phenylalkyl-C₁-C₄-Rest, wobei der Aromat gegebenenfalls durch Halogen, C₁-C₄-Alkyl-, Trifluormethyl-, Hydroxy-, C₁-C₄-Alkoxy-, Amino-, Cyano- oder Nitrogruppen substituiert ist, oder ein Phenylalkanon Rest bedeutet, wobei die Phenylgruppe durch Halogen substituiert sein kann,
R² eine gegebenenfalls durch Halogenatome, C₁-C₄-Alkyl, Trifluormethyl-, Trifluormethoxy-, Hydroxyl, C₁-C₄-Alkoxy-, Amino-, Monomethylamino-, Dimethylamin-, Cyano- oder Nitrogruppen mono- oder disubstituierte Phenyl-, Pyridyl-, Pyrimidinyl- oder Pyrazinyl-Gruppe darstellt, die gegebenenfalls mit einem Benzolkern, der gegebenenfalls durch Halogenatome, C₁-C₄-Alkyl-, Hydroxy-, Trifluormethyl-, C₁-C₄-Alkoxy-, Amino-, Cyano- oder Nitrogruppen mono- oder disubstituiert sein kann und gegebenenfalls 1 Stickstoffatom enthalten kann, oder mit einem 5- oder 6-gliedrigen Ring, der 1-2 Sauerstoffatome enthalten kann, anelliert sein kann,
- A: NH oder ein Sauerstoffatom darstellt,
- Y: CH₂, CH₂-CH₂, CH₂-CH₂-CH₂ oder CH₂-CH
ist,
Z ein Stickstoffatom, Kohlenstoffatom oder CH darstellt, wobei die Bindung zwischen Y und Z auch eine Doppelbindung sein kann,
und n die Zahl 2,3 oder 4 bedeutet
und deren Salze mit physiologisch verträglichen Säuren.

Weitere erfindungsgemäß brauchbare Verbindungen sind beispielweise in der DE 196 36 769.7 beschrieben. Hierbei handelt es sich um 3-substituierte 3,4,5,6,7,8-Hexahydro-pyrido[4',3':4,5]thieno-[2,3-d]pyrimidin-Derivate der Formel I 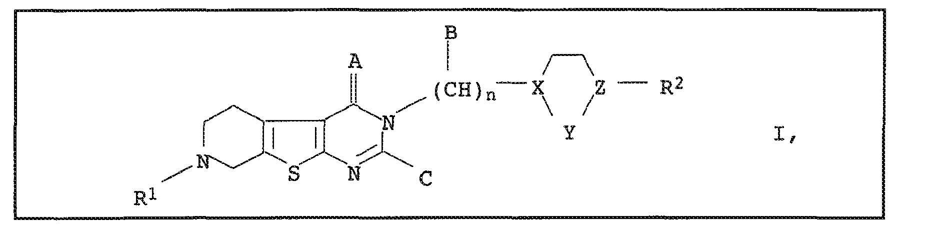 worin
R¹ ein Wasserstoffatom, eine C₁-C₄-Alkylgruppe, eine Acetyl- oder Benzoylgruppe, ein Phenylalkyl-C₁-C₄-Rest, wobei der Aromat gegebenenfalls durch Halogen, C₁-C₄-Alkyl-, Trifluormethyl-, Hydroxyl-, C₁-C₄-Alkoxy-, Amino-, Cyano- oder Nitrogruppen substituiert ist, ein NaphthylalkylC₁-C₃-Rest, ein Phenylalkanon-C₂-C₃-Rest oder ein Phenylcarbamoylalkyl-C₂-Rest bedeutet, wobei die Phenylgruppe durch Halogen substituiert sein kann,
R² eine gegebenenfalls durch Halogenatome, C₁-C₄-Alkyl, Trifluormethyl-, Trifluormethoxy-, Hydroxyl-, C₁-C₄-Alkoxy-, Amino-, Monomethylamino-, Dimethylamino-, Cyano- oder Nitrogruppen mono-, di- oder trisubstituierte Phenyl-, Pyridyl-, Pyrimidinyl- oder Pyrazinyl-Gruppe darstellt, die gegebenenfalls mit einem Benzolkern, der gegebenenfalls durch Halogenatome, C₁-C₄-Alkyl, Hydroxy-, Trifluormethyl, C₁-C₄-Alkoxy-, Amino-, Cyano- oder Nitrogruppen mono- oder disubstituiert sein kann und gegebenenfalls 1 Stickstoffatom enthalten kann, oder mit einem 5- oder 6-gliedrigen Ring, der 1-2 Sauerstoffatome enthalten kann, anelliert sein kann,
oder durch eine Phenyl-C₁-C₂-alkyl-bzw.-alkoxy-Gruppe substituiert sein kann, wobei der Phenylrest durch Halogen, eine Methyl-, Trifluormethyl- oder Methoxygruppe substituiert sein kann,
- A: NH oder ein Sauerstoffatom darstellt,
- B: Wasserstoff oder Methyl bedeutet,
- C: Wasserstoff, Methyl oder Hydroxy darstellt,
- X: ein Stickstoffatom bedeutet,
- Y: CH₂, CH₂-CH₂, CH₂-CH₂-CH₂ oder CH₂-CH ist,
- Z: ein Stickstoffatom, Kohlenstoffatom oder CH darstellt,
wobei die Bindung zwischen Y und Z auch eine Doppelbindung sein kann,
und n die Zahl 2, 3 oder 4 bedeutet,
und deren Salze mit physiologisch verträglichen Säuren.

Auch 5-HT5-spezifische Antikörper können als 5-HT5-Bindungspartner brauchbar sein. Es kann sich um polyklonale Antiseren, monoklonale Antikörper, Antikörperfragmente, wie F(ab), Fc, etc., chimäre und rekombinante Antikörper handeln. Die Herstellung solcher Antikörper kann in an sich bekannter Weise erfolgen. Als Immunogen kann man 5-HT5-Rezeptor als solchen oder antigene, in der Regel an übliche Trägerproteine gekoppelte Fragmente davon verwenden.

Weitere niedermolekulare 5-HT5-Bindungspartner, meist synthetische Verbindungen, sind in vielerlei Hinsicht vorteilhaft brauchbar.

Auch Aptamere, das sind Nukleinsäuren, in der Regel Oligonukleotide, mit ausreichender Affinität zu 5-HT5-Rezeptoren, können als Bindungspartner Anwendung finden.

Die erfindungsgemäße Anwendung ist nicht auf die vorstehend genannten Bindungspartner beschränkt. Vielmehr kann jede Substanz, die in der oben beschriebenen Art und Weise an 5-HT5-Rezeptoren bindet, erfindungsgemäß als 5-HT5-Bindungspartner Anwendung finden.

Assays zur Bestimmung von Bindungsaffinitäten von Testsubstanzen zu 5-HT-Rezeptoren sind im Prinzip bekannt. Dies kann beispielsweise dadurch geschehen, daß man die kompetitive Hemmung der Bindung eines Vergleichsbindungspartner an 5-HT5-Rezeptoren durch die zu untersuchende Substanz bewertet. Als Vergleichsbindungspartner eignen sich bekannte Liganden für 5-HT-Rezeptoren, wie 5-HT oder 5-CT oder LSD. Diese werden zweckmäßigerweise so markiert, daß sich deren Bindung an 5-HT-Rezeptoren analytisch mit Standardmethoden verfolgen läßt. Bevorzugt sind radioaktive und optische Markierungen. Bei Bindungsstudien an 5-HT5-Rezeptoren wird erfindungsgemäß 5-CT oder LSD insbesondere in Form von [³H]-LSD verwendet. Die Bindungsaffinitäten können als halbmaximale Hemmungkonstante IC₅₀ oder als Hemmkonstante Kᵢ ausgedrückt werden. Dieses Verfahren dient vorzugsweise zum primären Screening. Die SPA- oder die FlashPlate-Technologie kommt bevorzugt zur Anwendung.

Die Bindung zu untersuchender Bindungspartner kann man auch an 5-HT-Rezeptoren direkt bestimmen. Die Bindungsaffinität ausdrükkenden Hemmkonstanten Ki können beispielsweise kalorimetrisch, d.h. durch Messung der freigesetzten Bindungsenergie, bestimmt werden.

Auch Effektorfunktionen können mit Hilfe bekannter funktioneller Assays sowohl in vitro als auch in vivo qualitativ oder quantitativ bewertet werden.

Der Beurteilung einer agonistischen Aktivität können all diejenigen Effekte zugrundegelegt werden, die durch die Bindung von 5-HT an 5-HT5-Rezeptoren hervorgerufen werden. Erfindungsgemäß bevorzugt ist es, die Auswirkungen auf die Bindung von GTP an G-Proteine, auf interzelluläre Calcium-Spiegel, auf die Phospholipase C-Aktivität und/oder auf die cAMP-Produktion zu bewerten. Diese Verfahren dienen vorzugsweise zum sekundären Screening. Auch hier kommt die SPA- oder die FlashPlate-Technologie vorteilhaft zur Anwendung.

Die GTP-Bindung an G-Proteine kann untersucht werden, indem man ein nicht hydrolysierbares Analogon von GTP verwendet, beispielsweise [³⁵S]GTPγS, dessen Bindung radiologisch untersucht werden kann. Diese Untersuchung wird vorzugsweise an 5-HT5-Rezeptor aufweisenden Membranen durchgeführt.

Zur Messung intrazellulärer Calcium-Spiegel kann man geeignete Calcium-Sonden, in der Regel Calcium-Chelatoren, beispielsweise fluoreszierende Verbindungen, wie Fura-2-acetylmethylester oder Fluo-3-AM, einsetzen. Diese Untersuchung wird vorzugsweise an 5-HT5-Rezeptor aufweisenden Zellkulturen, insbesondere an Einzelzellen durchgeführt.

Die Phospholipase C-Aktvität läßt sich anhand der von ihr katalysierten Reaktionen bestimmen, beispielsweise dem Einbau von Myo-Inositol, das zu Nachweiszwecken vorzugsweise als [³H]-Myo-Inositol radioaktiv markiert ist, oder die Umsetzung von PPIP₂ zu IP₃, wobei auch das PPIP₂ vorzugsweise als [³²P]PIP₂ radioaktiv markiert ist. Diese Untersuchungen werden vorzugsweise an 5-HT5-Rezeptor aufweisenden Einzelzellen durchgeführt.

Die cAMP-Produktion kann mit Hilfe des cAMP-Bindungsproteins bestimmt werden. Diese Untersuchung wird vorzugsweise an 5-HT5-Rezeptor aufweisenden Einzelzellen durchgeführt.

Gegebenfalls bestimmt man auch die Effektorfunktion, d.h. die Aktivität von erfindungsgemäßen Bindungspartnern für andere 5-HT-Rezeptoren. Dies geschieht zweckmäßigerweise unter Berücksichtigung der für 5-HT5 und andere 5-HT-Rezeptoren ermittelten Bindungsaffinitäten, also insbesondere unter Berücksichtigung der Selektivität.

Gegenstand der vorliegenden Erfindung sind daher auch Verfahren zur Identifizierung und Charakterisierung erfindungsgemäß verwendbarer Bindungspartner. Diese und weitere in ähnlicher Weise geeignete Verfahren können die Grundlage bilden für in vitro-Screening-Verfahren, mit denen man aus einer Vielzahl verschiedener Verbindungen diejenigen auslesen kann, die im Hinblick auf eine künftige Anwendung am aussichtsreichsten zu sein scheinen. Beispielsweise können mittels kombinatorischer Chemie umfangreiche Stoffbanken angelegt werden, die Myriaden potentieller Wirkstoffe umfassen. Das Durchmustern kombinatorischer Substanzbibliotheken nach Stoffen mit gewünschter Aktivität ist automatisierbar. Screening-Roboter dienen der effizienten Auswertung der vorzugsweise auf Mikrotiterplatten angeordneten Einzelassays. So betrifft die vorliegende Erfindung auch Screening-Verfahren, d.h. sowohl Primär- als auch Sekundärscreening-Verfahren, bei denen vorzugsweise wenigstens eines der nachfolgend beschriebenen Verfahren zur Anwendung kommt. Kommen mehrere Verfahren zur Anwendung, so kann das zeitlich versetzt oder gleichzeitig an ein und derselben Probe oder an verschiedenen Proben einer zu untersuchenden Substanz geschehen.

Eine besonders effektive Technologie zur Durchführung derartiger Verfahren ist der im Bereich des Wirkstoffscreenings bekannte Scintillation Proximity Assay, kurz SPA genannt. Kits und Komponenten zur Durchführung dieses Assays können kommerziell bezogen werden, beispielweise bei Amersham Pharmacia Biotech. Im Prinzip werden solubilisierte oder membrangebundene Rezeptoren auf Scintillationssubstanz enthaltenden, kleinen Fluoromikrosphären immobilisert. Bindet beispielsweise ein Radioligand an die immobilisierten Rezeptoren, so wird die Scintillationssubstanz zur Lichtemission angeregt, da die räumliche Nähe zwischen Scintillationssubstanz und Radioligand gegeben ist.

Eine weitere besonders effektive Technologie zur Durchführung derartiger Verfahren ist die im Bereich des Wirkstoffscreenings bekannte FlashPlate^{R}-Technologie. Kits und Komponenten zur Durchführung dieses Assays können kommerziell bezogen werden, beispielweise bei NEN^{R} Life Science Products. Dieses Prinzip basiert ebenfalls auf Mikrotiterplatten (96er oder 384er), die mit Scintillationssubstanz beschichtet sind.

Die vorstehend genannten Assays sind dem Fachmann im Prinzip bekannt.

Ein erstes erfindungsgemäßes Verfahren dient zur Bestimmung der Affinität und/oder Selektivität von Bindungspartnern für 5-HT5-Rezeptoren. Zu diesem Zweck bringt man den Bindungspartner mit 5-HT5-Rezeptoren in Kontakt und bestimmt die Bindungsaffinität.

Zur Bestimmung von Selektivitäten bestimmt man in gleicher Weise - gegebenenfalls unter Verwendung der für den jeweiligen Rezeptor spezifischen Liganden - die Bindungsaffinität der zu untersuchenden Bindungspartner an andere 5-HT-Rezeptoren und vergleicht die erhaltenen Werte.

Ein weiteres erfindungsgemäßes Verfahren betrifft die Bestimmung der Aktivität von Bindungspartnern für 5-HT5-Rezeptoren, d.h. die Bestimmung agonistischer, teilagonistischer, antagonistischer und/oder teilantagonistischer Wirkung. Zu diesem Zweck bringt man den Bindungspartner mit 5-HT5-Rezeptoren in Kontakt und bewertet die durch die Bindung hervorgerufenen Effekte.

Gemäß einer bevorzugten Ausführungsform werden Bindungspartner einem primären Screening unterzogen, indem man ihre Bindungsaffinität zu 5-HT5-Rezeptoren mit dem oben beschriebenen [³H]-5-CT- oder [³H]-LSD-Kompetitionsexperiment bestimmt. Diejenigen Bindungspartner, die eine Hemmkonstante IC₅₀ im Bereich von 10⁻⁶ M oder weniger aufweisen, werden dann einem sekundären Screening unterzogen, indem man ihre Effektorfunktion insbesondere im Hinblick auf die GTP-Bindung und/oder die intrazellulären Calcium-Spiegel in der oben beschriebenen Weise bewertet. Schließlich können die so ausgewählten Bindungspartner zur Selektivitätbestimmung einem Gegen-Screening unterzogen, indem man ihre Bindungsaffinität zu weiteren 5-HT-Rezeptoren im wesentlichen in der vor-stehend beschriebenen Art und Weise - jedoch gegebenenfalls unter Verwendung der für den jeweiligen Rezeptor spezifischen Liganden - bestimmt. Beispielsweise kann man [³H]-8-Hydroxy-di-propylamino-tetralin ([³H]-8-DPAT) für Bindungsstudien an 5-HT1A-Rezeptoren verwenden, während 5-HTIB- und 5-HT1D-Rezeptoren mit [³H]-5-CT untersucht werden können.

5-HT5-Rezeptoren werden vorzugsweise in Form zellulärer Systeme zur Verfügung gestellt, d.h. in Form von Membranen, Zellen, Zellverbänden, Geweben oder Organen, die 5-HT5-Rezeptoren tragen. Derartige zelluläre Systeme können 5-HT5-Rezeptoren von Natur aus exprimieren, sie können aber auch durch geeignete genetische Manipulation, z.B. durch Transfektion, zur 5-HT5-Expression veranlaßt sein. Im Rahmen der h5-HT5 betreffenden bevorzugten Ausführungsform der vorliegenden Erfindung kann man hierzu insbesondere die in Rees S. et al, FEBS Letters 335:242-246 (1994) beschriebenen kodierende Sequenz verwenden (Zugangsnummer X81411). Humane Gliom-Zellinien werden als natürliche, 5-HT5-Rezeptoren aufweisende zelluläre Systeme bevorzugt. Von den h5-HT5-transfizierten heterologen Zellinien werden diejenigen bevorzugt, die das h5-HT5-Gen stabil exprimieren. Zu nennen sind beispielsweise h5-HT5-transfizierte CHO-Zellen, h5-HT5-transfizierte humane Nierenzellen, insbesondere h5-HT5-transfizierte HEK293-Zellen, oder h5-HT5-transfizierte C-6-Gliomzellen.

Zur Bestimmung von Selektivität, Affinität und Aktivität erfindungsgemäßer Bindungspartner können auch Gehirngewebeschnitte und native Membranen aus Gehirnteilen verwendet werden. Werden radioaktive Marker eingesetzt, so erfolgt die Auswertung von Gewebeschnitten vorzugsweise autoradiographisch.

Die neuroprotektive Wirkung wird vorzugsweise an Tiermodellen für neurodegenarative und neuropsychiatrische Vorgänge bestimmt.

Bevorzugt sind Tiermodelle für Gehirnschlag und cerebrale Störungen bei Mehrfach-Infarkt, beispielsweise cerebrale Ischämien infolge eines Verschlusses der Halsschlagader oder der mittleren Cerebralarterie (MCA-Okklusion), Vorderhirnischämien und Hypoxietoleranztests, anxiolytische Modelle, beispielsweise Wirstoff-induzierte Konvulsionen, Elektroschock- oder Isolations-induzierte Aggressionen, Modelle für antiepileptische Aktivität, beispielsweise Elektroschock-, Wirkstoff- oder Schall-induzierte Anfälle und genetische Modelle, excitotoxische Neurodegenerationsmodelle und Demyelinisationsmodelle.

Die erfindungsgemäße Verwendung von 5-HT5-Bindungspartnern beinhaltet im Rahmen der Behandlung ein Verfahren. Dabei wird dem zu behandelnden Individuum, vorzugsweise einem Säuger, insbesondere einem Menschen, Nutz- oder Haustier, eine wirksame Menge eines oder mehrerer 5-HT5-Bindungspartner, in der Regel der pharmazeutischen und tierarzneilichen Praxis entsprechend formuliert, verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken, bestimmte Anzeichen, Symptome und/oder Fehlfunktionen zu entwickeln, und weitere Faktoren miteinbezieht.

Die Behandlung erfolgt in der Regel durch einmalige oder mehrmalige tägliche Verabfolgung gegebenenfalls zusammen oder im Wechsel mit anderen Wirkstoffen oder wirkstoffhaltigen Präparaten, so daß einem zu behandelnden Individuum eine Tagesdosis von etwa 0,001 g bis 10 g, vorzugsweise von etwa 0,001 g bis etwa 1 g zugeführt wird.

Die Erfindung betrifft auch die Herstellung pharmazeutischer Mittel zur Behandlung eines Individuums, vorzugsweise eines Säugers, insbesondere eines Menschen, Nutz- oder Haustieres. Die erfindungsgemäßen Bindungspartner werden gewöhnlich in Form von pharmazeutischen Zusammensetzungen verabreicht, die einen pharmazeutisch verträglichen Exzipienten mit wenigstens einem erfindungsgemäßen Inhibitor und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Bindungspartner verwendet werden. Ferner können auch Liposomen, Mikrosphären oder Polymermatrizes zur Anwendung kommen.

Bei der Herstellung der Zusammensetzungen werden erfindungsgemäße Bindungspartner gewöhnlich mit einem Exzipienten vermischt oder verdünnt. Exzipienten können feste, halbfeste oder flüssige Materialien sein, die als Vehikel, Träger oder Medium für den Wirkstoff dienen.

Zu geeigneten Exzipienten gehören beispielsweise Lactose, Dextrose, Sucrose, Sorbitol, Mannitol, Stärken, Akaziengummi, Calciumphosphat, Alginate, Traganth, Gelatine, Calciumsilikat, mikrokristalline Cellulose, Polyvinylpyrrolidon, Cellulose, Wasser, Sirup und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Die vorliegende Erfindung wird anhand der nachfolgenden Beispiele näher erläutert, ohne darauf beschränkt zu sein.

### Referenzbeispiel 1

### h5-HT5-Rezeptor exprimierende HEK293-Zellen und CHO-Zellen

Das für den humanen 5-HT5-Rezeptor kodierende Gen wurde in an sich bekannter Weise über 3'-5'-RT-PCR (RACE-System, Boehringer Mannheim) aus menschlichen Geweben isoliert. Die Gensequenz wurde dann in ein das Neomycinresistenz-Gen tragendes Plasmid insertiert (pcDNA3; Invitrogen, Deutschland) und in E. coli den Herstellerhinweisen entsprechend amplifiziert. Eine Präparation des resultierenden Plasmids wurde mit Lipofectamin^{R} (Gibco Life-Sciences, Deutschland) vermischt, und HEK293-Zellen wurden in Petrischalen (2,5 cm) mit einer dünnen Schicht dieses Transfektionsgemisches inkubiert. Danach wurde das Transfektionsgemisch durch Neomycin-haltiges Kulturmedium ersetzt. Überlebende Zellen wurden weiter in DMEM-F12-Medium kultiviert, das mit 10% fötalem Kälberserum, 2 mM Glutamin und Antibiotika (90 mg Streptomycin, 90 mg Penicillin) ergänzt war. Die Zellen wurden bei 5% CO₂, 95% Luftfeuchtigkeit und 37°C bis zur Konfluenz aufgezogen.

In analoger Weise erhält man h5-HT5-Rezeptor exprimierende CHO-Zellen.

### Referenzbeispiel 2

### Zellmembran-Präparation

Die hier verwendete Methode lehnt sich im wesentlichen an bekannte Methoden zur Präparation von Zellmembranen aus Zellen an (Findlay J.B.C. und Evans W.H. Biological Membranes, Practical Approach (1987)). Die gemäß Referenzbeispiel 1 kultivierte Zellen wurden vorsichtig von der Oberfläche des Kulturgefäßes abgeschabt und 10 min bei 180xg in DMEM-F12-Medium zentrifugiert. Die gewonnenen Zellpellets wurden in 5 mM EDTA, 5 mM EGTA, 0,1 mM PMSF und 3 mM Benzamidin enthaltendem 5 mM Tris-HCl-Puffer (pH: 7,6; Puffer A) resuspendiert und 15 min bei 4°C inkubiert. Die Zellsuspension wurde in einem Ultraturrax^{R} (15000 UPM) homogenisiert (6x3s) und 1 min bei 1000xg und 4°C zentrifugiert. Das Pellet wurde in Puffer A resuspendiert und, wie vorstehend beschrieben, homogenisiert und zentrifugiert. Die Überstände aus beiden Schritten wurden gesammelt und 20 min bei 40000xg und 4°C zentrifugiert. Das Pellet wurde in Puffer A resuspendiert und homogenisiert (1x15s). Die Membransuspension wurde 20 min bei 40000xg und 4°C zentrifugiert. Das resultierende Pellet wurde in 10% Glycerin und 1% Rinderserumalbumin enthaltendem Puffer A resuspendiert. Es wurden Aliquots eingefroren und bei -80°C bis zum Gebrauch aufbewahrt.

### Referenzbeispiel 3

### Kinetik der Sättigungsbindung von [³H]-5-CT

Die Methodik ist im wesentlichen bekannt (Ress S. et al., FEBS Letters 335:242-246 (1994)). Gemäß Referenzbeispiel 2 gewonnene Membranen (200 µl) wurden bei einem Gesamtvolumen von 600 µl in 1 mM EDTA enthaltendem 100 mM Tris-HCl (pH: 7,7; Puffer B) mit ansteigenden Konzentrationen an [³H]-5-CT (96 Ci/mmol) inkubiert, wobei zur Bestimmung der spezifischen Bindung 10 µM Methiothepin zugesetzt wurde, während zur Bestimmung der Gesamtbindung Methiothepin nicht zugesetzt wurde. Es wurde 90 min bei 30°C inkubiert. Danach wurden die Proben filtriert, wobei man ein Skatron^{R}-Filtrationssystem und in 0,3% Polyethylenimid eingebettete GF/B-Filter verwendete. Die Filter wurden mit 9 ml Puffer B bei 4°C gewaschen. Die auf den Filtern zurückgehaltene Radioaktivität wurde mittels Flüssigscintillationszählung gemessen, wobei man 5 ml Ultima-Gold (Packard) verwendete.

### Referenzbeispiel 4

### a) [³H]-5-CT-Bindungskompetition

Die Experimente zur Bindungskompetition erfolgten im wesentlichen in Anlehnung an bekannte Untersuchungen (Rees et al., 1994). Gemäß Beispiel 2 gewonnene Membranen (200 µl) wurden in einem Gesamtvolumen von 600 µl in Puffer B mit ansteigenden Konzentrationen ausgewählter Verbindungen in Gegenwart von 2 nM [³H]-5-CT inkubiert. Nach einer Inkubationszeit von 75 min bei 30°C wurden die Proben mit Puffer B bei 4°C über in 0,3% Polyethylenimid eingebetteten GF/B-Filtern filtriert. Die Filter wurden mit 9 ml Puffer B gewaschen. Die auf den Filtern zurückgehaltene Radioaktivität wurde wie in Referenzbeispiel 3 bestimmt. Die Gesamtbindung wurde definiert als diejenige Bindung des Radioliganden, die ohne Zusatz weiterer Verbindungen beobachtet wurde. Die nicht-spezifische Bindung wurde definiert als diejenige Bindung von [³H]-5-CT, die in Gegenwart von 10 µM Methiothepin beobachtet wurde. Es können auch ähnliche Systeme verwendet werden, die durch Verwendung von Mikrotiterplatten einen hohen Probendurchsatz und ein Sekundärscreening gestatten.

Die Sättigungsparameter der [³H]-5-CT-Bindung wurden sowohl durch nicht-lineare Regressionsanalyse als auch aus linearen Auftragungen bei Verwendung der SigmaPlot-Software (Jandel Scientific, Germany) bestimmt. Es wurden Kompetitionskurven aufgestellt, in denen die radioaktive Bindung als prozentualer Anteil der Gesamtbindung ausgedrückt ist. Halbmaximale Hemmkonstanten IC₅₀ und Hill-Koeffizienten (n_{H}) wurden mittels nicht-linearer Regressionsanalyse ermittelt.

### b) Identifizierung von h5-HT5-Rezeptorliganden durch HTS unter Verwendung der FlashPlate-Technologie.

96-Well-FlashPlates, die mit h5-HT5-Membranen beschichtet sind, können von Bio Signal Inc. (Kanada) bezogen werden. [3H]-LSD wurde in Tris-HCl-Puffer, der 10 mM MgCl₂, 0,5 mM EDTA und 0,5% BSA enthält, auf eine geeignete Konzentration verdünnt. Die Radioligand-Lösung wurde in die Wells (25 ml) gegeben, die entweder Testverbindung enthielten oder nicht. Die Platten wurden 180 Minuten bei Raumtemperatur inkubiert und das radioaktive Signal wurde mit einem Mikro-β-Zähler (Wallac) gemessen. Die nicht spezifische Bindung wurde mit Methiothepin bestimmt. [3H]-LSD besitzt eine Affinität von 12 nM. Mit zunehmender Bindungsaffinität der Testverbindung nahm das radioaktive Signal von [3H]-LSD ab.

### Referenzbeispiel 5

### Bestimmung der Agonist-induzierten Stimulierung der [³⁵S]GTP-yS-Bindung

[³⁵S]GTPγS-Bindungsassays sind bekannt. Der vorliegende Assay wurde in Anlehnung an die zuvor beschriebene Methode von Hilf, G. und Jakobs, K.H. (Eur. J. Mol. Pharmacol. 225:245-252 (1992)) durchgeführt. Es wurden Wirkstoff-induzierte Veränderungen der [³⁵S]GTPγS-Bindung an Membranen aus stabil mit dem h5HT5-Rezeptorgen transfizierten HEK293-Zellen gemessen (siehe Referenzbeispiele 1 und 2). Die Zellmembranen (12 µg) wurden mit 6,75 mM MgCl₂, 150 mM NaCl, 1 mM DTT, 1 mM EDTA, 10 µM GDP und [³⁵S]GTPγS enthaltendem 50 mM Triethanolamin-HCl-Puffer (pH: 7.5) inkubiert. Im Anschluß an eine 60-minütige Inkubation bei 30°C mit oder ohne Zusatz der zu testenden Wirkstoffe wurde das Testgemisch (100 µl) rasch über GF-B-Filtern bei Verwendung einer Skatron^{R}-Filtrationsvorrichtung filtriert. Die Filter wurden rasch mit 100 mM NaCl und 5 mM MgCl₂ enthaltendem 50 mM Tris-HCl-Puffer (9 ml; pH: 7,5; 4°C) gewaschen. Die auf den Filtern zurückgehaltene Radioaktivität wurde mittels Scintillationsspektrometrie bestimmt, wobei man Ultima Gold-Scintillationsflüssigkeit verwendete. Ebenfalls verwendet werden können ähnliche Systeme, die durch Verwendung von Mikrotiterplatten einen hohen Durchsatz und ein Sekundärscreening gestatten.

Die Wirkstoffaktivitäten wurden als prozentualer Anteil der in Abwesenheit des Wirkstoffes gemessenen Grundbindung ausgedrückt. Die Anpassung der Kurven erfolgte mit einer Software zur nichtlinearen Regressionsanalyse (SigmaPlot, Jandel Scientific, Deutschland) gemäß der allgemeinen Gleichung E=(L*Eₘₐₓ)/(L+EC₅₀), worin E die Wirkung, L die Ligandkonzentration, Eₘₐₓ die Maximalwirkung und EC₅₀ diejenige Konzentration, die 50% der Maximalwirkung induziert, bedeutet.

### Referenzbeispiel 6

### Bestimmung Agonist-induzierter Veränderungen intrazellulärer Calciumspiegel

Die Methode ist bekannt (Kao J.P.Y. Methods in Cell Biology 40:155-181 (1994)). Wie in Referenzbeispiel 1 beschrieben, wurden h5HT5-Rezeptor exprimierende HEK293-Zellen in Kulturgefäßen aufgezogen. Die Zellen wurden vorsichtig abgeschabt, bevor sie konfluent waren. Die Zellen wurden mit Fura-2 markiert, indem man mit Fura-2-acetylmethylester (Sigma) bei Raumtemperatur inkubierte. Die Zellen wurden bei 180xg 10 min zentrifugiert und in DMEM-F12-Medium ohne Serum resuspendiert und bei 37°C, 5% CO₂ und 95% Luftfeuchtigkeit 45 min inkubiert.

Intrazelluläre Calciumspiegel wurden mit einem Fluoreszenzmikroskop bestimmt, das mit einem geeigneten Filteraustauschsystem ausgestattet war (Olympus/Hamamatsu). Es wurde das Fluoreszenzverhältnis (340 nm / 380 nm) mit der ArguS^{R}-Software bestimmt. Die intrazellulären Calciumspiegel wurden über kurze Zeit in einzelnen Zellen ohne den Zusatz von Wirkstoffen und dann 30 min nach Zugabe des zu testenden Wirkstoffs beobachtet. Ebenfalls verwendet werden können ähnliche Systeme, die durch die Verwendung von Mikrotiterplatten einen hohen Durchsatz und ein Sekundärscreening gestatten.

In analoger Weise kann die Modulierung intrazellulärer Ca²⁺-Spiegel im HTS bewertet werden. Dazu wurden h5-HT5-Rezeptor exprimierende CHO-Zellen über Nacht in 96-Well-Platten kultiviert (30.0000 - 80.000 Zellen/Well). Die Zellen wurden eine Stunde mit 1 mM Fluo-3-AM, 10% Pluronsäure und 2,5 mM Probencid enthaltendem HEPES-Puffer markiert und gewaschen. Zu jedem Well wurde eine Testverbindung gegeben. Zur Bestimmung der Calciumspiegel wurde die Fluoreszenz-Intensität mit einem fluorometrisch arbeitendem Plattenlesegerät (Fluorometric Imaging Plate Reader; FLIPR) abgelesen.

### Referenzbeispiel 7

### Bestimmung der Agonist-induzierten Phospholipase C-Aktivität

Die Methode ist im wesentlichen bekannt (Garcia-Ladona F.J. et al., Neuroreport 4:691-694 (1993)). Die Zellen wurden 24 h mit 0,125 µM [³H]Myo-Inositol inkubiert. Nicht eingebautes [³H]Myo-Inositol wurde aus dem Medium entfernt und durch 10 mM LiCl enthaltendem Krebs-Henseleit-Puffer ersetzt. Nach 10-minütiger Inkubation wurde der zu testende Wirkstoff zugesetzt. Nach 45 min wurde die Reaktion gestoppt, indem man das Stimulationsmedium durch destilliertes Wasser ersetzte. Verwendet man Gewebeproben, geht man in ähnlicher Weise vor (Garcia-Ladona et al., 1993). Die Zellen wurden eingefroren und bei -80°C gelagert. Es wurde die Produktion von [³H]Inositolmonophosphat mittels bekannter chromatographischer Methoden bestimmt. Eine ähnliche Methode kann mit Gewebe-Miniprismen zur Anwendung kommen. Die Bestimmung der Phospholipase C-Stimulierung wurde ebenfalls in ähnlicher Weise durchgeführt, indem man Membranfraktionen, wie in Referenzbeispiel 2 beschrieben, präparierte und mit [³²P]PIP₂ und Wirkstoffen inkubiert. In diesem Fall wurde die Produktion von IP3 bestimmt. Es wurden auch bekannte Verfahren optimiert, um auf Mikrotiterplatten basierende Systeme zu verwenden. Kommerziell erhältliche Materialien gestatten die Ausdehnung auf Analysen mit hohem Durchsatz und die Durchführung eines Sekundärscreenings.

### Referenzbeispiel 8

### Bestimmung der Agonist-induzierten Veränderung der cAMP-Produktion

Die verwendete Methode ist im wesentlichen bekannt (Strada S.S. et al., Methods in Neurotransmission receptor analysis: 89-110 (1990)). Zellen wurden 10 min in Kulturmedium ohne Serum und Antibiotika inkubiert. Es wurde 15 min auf 95°C erwärmt, um die Reaktion zu stoppen. Die Zellproben wurden eingefroren und bei -80°C gelagert. cAMP-Spiegel wurden mit kommerziell erhältlichen Kits bestimmt, die das cAMP-Bindungsprotein verwenden. Es wurden auch bekannte Verfahren optimiert, um auf Mikrotiterplatten basierende Systeme zu verwenden. Kommerziell erhältliche Materialien gestatten die Ausdehnung auf Analysen mit hohem Durchsatz und die Durchführung eines Sekundärscreenings.

### Referenzbeispiel 9

### Gewebepräparation

90 min nach Verabreichung des Wirkstoffs (oral, intraperitoneal, intravenös oder intracerebroventriculär) wurden die Versuchstiere enthauptet. Das gesamte Gehirn wurde rasch aus dem Schädel entfernt, auf Trockeneis gefroren und bei -80°C gelagert. Rattenhirnschnitte (15 µm) wurden bei -20°C in einem Cryostat erhalten, auf Gelatine-beschichtete Objektträger aufgebracht und bei -30°C bis zum Gebrauch gelagert.

### Referenzbeispiel 10

### Neuroprotektive Wirkung: MCA-Okklusion

Die neuroprotektive Wirksamkeit der Testverbindungen wurde an einem Standardmodell zum experimentell verursachten Gehirnschlag untersucht. Die Experimente wurden an männlichen Long Evans-Ratten vorgenommen. Unter Lachgas-unterstützter Halothan-Anästhesie wurde die mittlere Cerebralarterie (MCA) durchtrennt und an distaler Position, wie in der Literatur beschrieben, dauerhaft ligiert. Es wurde das resultierende Infarktvolumen 22 Stunden nach MCA-Okklusion bestimmt.

### Referenzbeispiel 11

### Neuroprotektive Wirkung: experimentelles Hirntrauma der Ratte

Die laterale "fluid-percussion"-Methode (McIntosh TK, Neuroscience 28, 233 244, 1989) wurde verwendet, um an der Ratte ein experimentelles Hirntrauma zu erzeugen, welches zur Testung potentieller neuroprotektiver Substanzen geeignet ist. Mittels dieser Technik wurde eine kontrollierte und konsistente Gewebeschädigung unterhalb der Aufschlagstelle erzeugt, welche den Neocortex, den Hippocampus und den Thalamus umfaßte.

An der narkotisierten Ratte wurde die rechte temporo-parietale Region des Schädels freigelegt. Der Schädel wurde über dem parietalen Cortex trepaniert (ca. 4 mm Durchmesser), wobei die Dura intakt gelassen wurde. Über der Trepanation wurde ein Luer-Lock-Aufsatz mit Zahnzement auf dem Schädel befestigt. Nach dem Aushärten des Zahnzementes wurde die Ratte mit der "fluid-percussion"-Apparatur nach McIntosh verbunden. Diese bestand aus einem mit 0,9 % NaCl-Lösung gefüllten Zylinder, wobei ein Ende des Zylinders mit einem Kolben verschlossen war, während das andere Ende über einen Druckaufnehmer mit einem Luer-Lock-Ansatz verbunden war. Mit diesem wurde das Gegenstück an der Ratte dicht verschlossen, so daß eine an der Dura anliegende Flüssigkeitssäule gebildet wurde. Von einer vorbestimmten Höhe ließ man ein Metallpendel auf den Kolben aufschlagen, so daß ein kurzer Schlag der komprimierten Flüssigkeitssäule auf die Oberfläche des Rattenhirns auftraf. Es wurden hohe Drucke von ca. 2,5 - 2,9 bar angewendet.

Die Ratten wurden 14 Tage nach der Traumatisierung zur Gehirnentnahme getötet. Nach Fixation der Gehirne wurden im Gefriermikrotom Schnitte von 30 mm Dicke hergestellt und mit Toluidin-Blau gefärbt. Zur Quantifizierung der Neuronenschädigung wurden Zellzählungen beiderseits im rostralen Hippocampus durchgeführt; es wurde der Anteil intakter Zellen im Gyrus dentatus auf der traumatisierten (rechten) sowie der (linken) Gegenseite ermittelt. Angegeben wurde der Quotient aus der Zellzahl im ipsilateralen und der Anzahl im contralateralen Hippocampus.

### Referenzbeispiel 12

### Synthese getesteter Bindungspartner

### a) 3,4,5,6,7,8-Hexahydro-7-methyl-3-[2-(4-(3-trifluormethyl-phenyl)-piperazin-1-yl)-ethyl]-pyrido[4',3':4,5]thieno[2,3-d]-pyrimidin-4-on x 2HCl (Verbindung A)

Die Verbindung wurde hergestellt, indem man 2-Ethoxymethylen-amino-3-cyano-6-methyl-4,5,6,7-tetrahydro-thieno[2,3-c]pyridin oder 2-Ethoxymethylen-amino-3-carboethoxy-6-methyl-4,5,6,7-tetrahydro-thieno[2,3-c]pyridin mit 1-(2-Amino-ethyl)-4-(3-trifluormethylphenyl)-piperazin in einem inerten Lösungsmittel wie Ethanol unter Rückfluß erhitzte. Das Reaktionsgemisch wurde aufgearbeitet, und das Produkt mit einem Schmelzpunkt von 309-312 °C wurde durch Ausfällen mit etherischer Salzsäure ausgefällt.

Eine weitere Möglichkeit zur Herstellung bestand darin, die oben genannten Ausgangsverbindungen zunächst mit Ethanolamin in einem inerten Lösungsmittel wie Ethanol unter Rückfluß zu erhitzen. Nachdem man das Reaktionsgemisch aufgearbeitet hatte, wurde das erhaltene 3,4,5,6,7,8-Hexahydro-3-(2-hydroxy)-ethyl-7-methyl-pyrido[4',3':4,5]thieno[2,3-d]pyrimidin-4-on beispielsweise mit Thionylchlorid umgesetzt, um eine geeignete Abgangsgruppe einzuführen, so daß die Umsetzung des resultierenden 3,4,5,6,7,8-Hexahydro-3-(2-chlor)-ethyl-7-methyl-pyrido-[4',3':4,5]thieno[2,3-d]pyrimidin-4-on mit N-(3-Trifluormethylphenyl)-piperazin in einem inerten Lösungsmittel wie Xylol unter basischen Bedingungen (z.B. Kaliumcarbonat) nach Aufarbeitung des Reaktionsgemisches das erwünschte Produkt ergab.

### b) 3,4,5,6,7,8-Hexahydro-6-ethyl-3-[2-(4-(1-naphthyl)-piperazin-1-yl)-ethyl]-pyrido[3',4':4,5]thieno[2,3-d]-pyrimidin-4-on x 3 HCl x 2H₂O (Verbindung B)

Die Herstellung erfolgte im Prinzip wie für Verbindung A beschrieben.

2-Ethoxymethylen-amino-3-carboethoxy-5-ethyl-4,5,6,7-tetrahydrothieno[3,2-c]pyridin wurde mit 1-(2-Amino-ethyl)-4-(1-naphthyl)-piperazin in einem inerten Lösungsmittel wie Ethanol unter Rückfluß erhitzt. Das Reaktionsgemisch wurde aufgearbeitet, und das in das Hydrochlorid überführte Produkt mit einem Schmelzpunkt von 298-300 °C wurde isoliert.

Alternativ wurde 3-(2-Chlor-ethyl)-6-ethyl-3,4,5,6,7,8-hexahydropyrido[3',4':4,5]thieno[2,3-d]pyrimidin-4-on mit N-(1-Naphthyl)-piperazin in einem inerten Lösungsmittel wie Xylol unter basischen Bedingungen umgestzt.

### c) 3,4,5,6,7,8-Hexahydro-7-methyl-3-[2-(4-(7-methoxynaphth-1-yl)-piperazin-1-yl)-ethyl]-pyrido[4',3':4,5]thieno[2,3-d]-pyrimidin-4-on x 2 HCl x H₂O (Verbindung C)

Die Herstellung kann analog Verbindung A erfolgen.

Verbindung A, B und C sowie die zu deren Herstellung benötigten Zwischenprodukte sind bekannt oder lassen sich gemäß den in der Literatur beschriebenen Herstellungsmethoden aus analogen Ausgangsmaterialien synthetisieren (F. Sauer und P. Stanetty, Monatsh. Chem. (1975), 106(5), 1111-1116; K. Gewald et al, Chem. Ber. 99, 94-100 (1966), Patentanmeldungen DE 196 36 769.7 und der DE 197 24 979.5).

### Beispiel 1

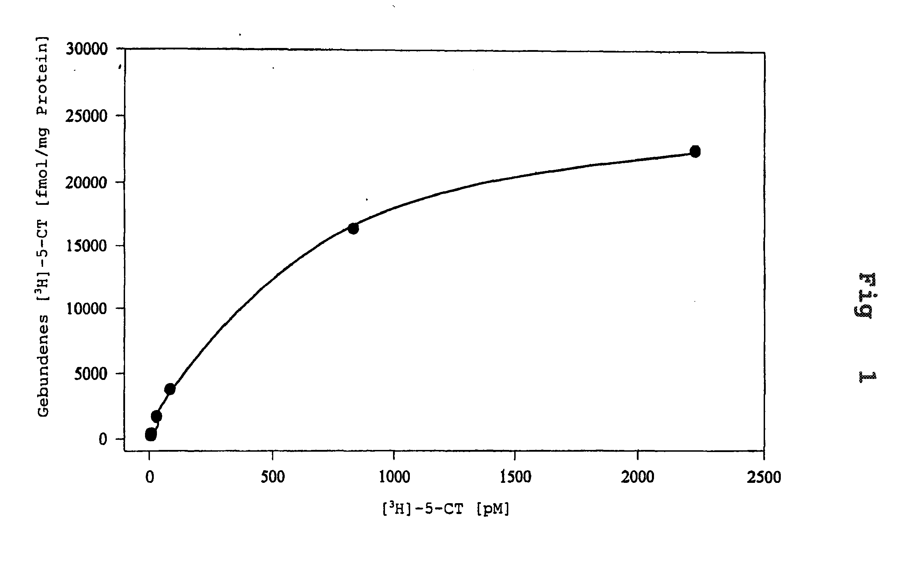

Gemäß Referenzbeispiel 3 wurde die Bindungsaffinität von [³H]-5-CT an 5-HT5-Rezeptoren bestimmt. Figur 1 zeigt eine Auftragung von gebundenem [³H]-5-CT in Abhängigkeit der [³H]-5-CT-Konzentration. Es wurde eine Dissoziationskonstante von K_{d} = 0.570 nM bestimmt. Die Rezeptorbindungsdichte (B) variierte je nach klonaler Zellinie in einem Bereich von 900-28000 fmol/mg Protein.

### Beispiel 2

Gemäß Referenzbeispiel 4 wurden die Bindungsaffinitäten serotoninerger Verbindungen anhand der [³H]-5-CT-Bindungskompetition bestimmt. Anhand der erhaltenen IC₅₀-Werte wurden die Hemmkonstanten Ki folgender Verbindungen bestimmt (Ki = IC₅₀/(1+C/K_{d})), wobei C die Konzentration an [³H]-5-CT ist und K_{d} gemäß Beispiel 1 bestimmt wurde):

| Verbindung | Ki [M] |
|---|---|
| R(+)-8-OH-DPAT | 1,25·10⁻⁷ |
| 5-CT | 1,44·10⁻⁹ |
| Verbindung A | 6,61·10⁻⁷ |
| Verbindung B | 1,24·10⁻⁷ |
| Verbindung C | 2,37·10⁻⁷ |

### Beispiel 3

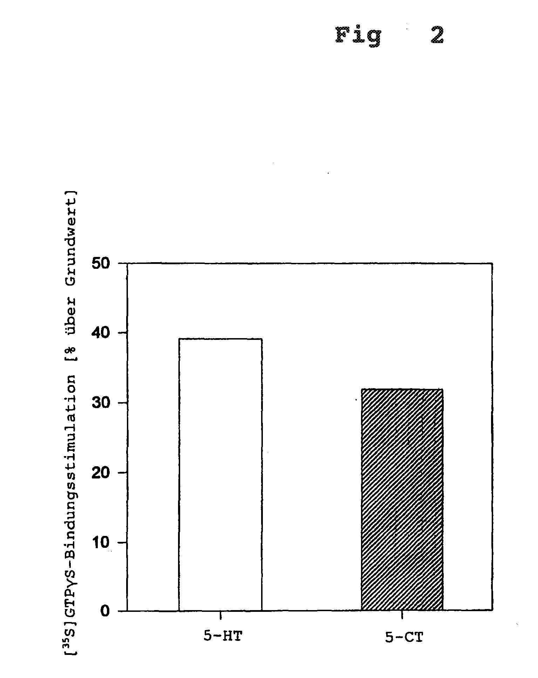

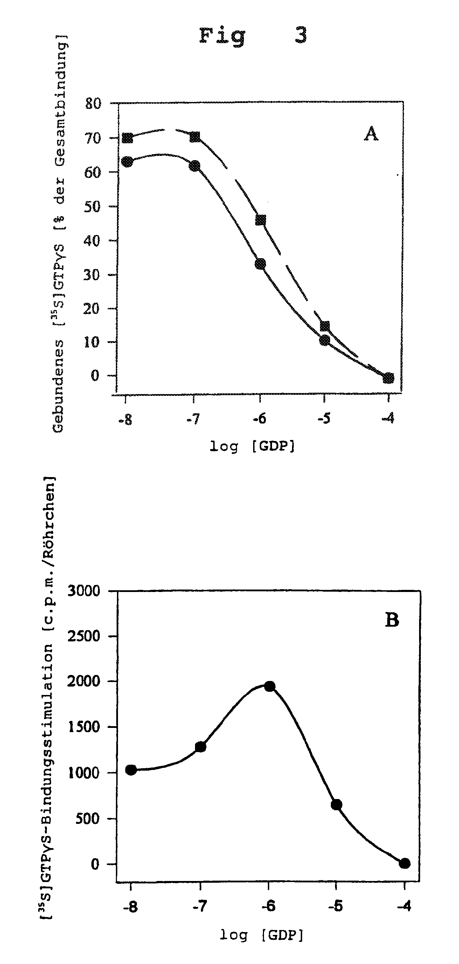

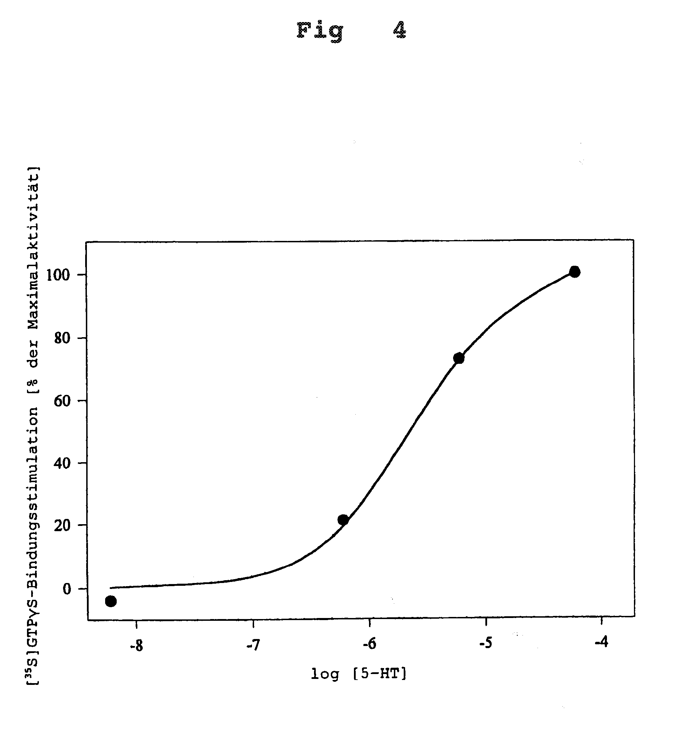

Gemäß Referenzbeispiel 5 wurde die Wirkstoff-induzierte Bindung von GTP an G-Proteine untersucht. Die Kopplung von 5-HT5-Rezeptoren an G-Proteine in HEK293-Zellen war evident. Die typischen serotoninergen Agonisten 5-HT und 5-CT induzierten einen Anstieg der [³⁵S]GTPγS-Bindung an die Zellmembranen von über 40 % über dem Grundwert (siehe Figur 2). Der 5-HT5-Rezeptor benötigt GDP für die durch Agonisten vermittelte Kopplung an G-Proteine (siehe Figur 3A). Der 5-HT-Effekt war dosisabhängig (siehe Figur 4) mit einer EC₅₀ von 2,6 µM.

### Beispiel 4

Gemäß Referenzbeispiel 10 wurde die neuroprotektive Wirkung ausgewählter 5-HT5-Bindungspartner getestet. Die Wirkstoffe wurden 90 Minuten nach MCA-Okklusion intravenös zunächst als Bolus und anschließend als Erhaltungsinfusion verabreicht. Folgende Resultate wurden erzielt:

| Wirkstoff | Dosis, i.v. [mg/kg + mg/kg/h] | Infarktvolumen, % der Kontrolle [Mittelwert ± SD, (n)] | | P, Student-t-Test, zweiseitig |
|---|---|---|---|---|
| | | Placebo | Wirkstoff | |
| R(+)-8-OH-DPAT | 2 + 1 | 100 ± 11 (12) | 72 ± 32 (12) | 0,0086 |
| Verbindung A | 4 + 2 | 100 ± 25 (12) | 62 ± 18 (10) | 0,0013 |
| Verbindung B | 2 + 1 | 100 ± 20 (12) | 66 ± 19 (9) | 0,0015 |
| Verbindung C | 1 + 0,5 | 100 | 67 | < 0,05 |

### Beispiel 5

Gemäß Referenzbeispiel 11 wurde ein experimentelles Hirntrauma der Ratte mit der lateralen "fluid-percussion"-Methode induziert. Die protektive Wirkung der getesteten Wirkstoffe auf das Überleben hippokampaler Neurone ist ausgedrückt als Quotient der Neuronenzahl der Traumaseite zur Neuronenzahl der kontralateralen Seite; angegeben ist jeweils der Mittelwert (MW) und der mittlere Fehler des Mittelwertes (sₘ)

| Wirkstoff | Dosis [mg/kg i.p.] | Quotient MW ± sₘ (n) | | P, Student-t-Test, zweiseitig |
|---|---|---|---|---|
| | | Placebo | Wirkstoff | |
| Verbindung C [15 + 120 min post] | 20 + 20 | 0,33 ± 0,05 (10) | 0,56 ± 0,05 (10) | < 0,05 |
| Verbindung B [15 + 120 min post] | 20 + 20 | 0,44 ± 0,03 (17) | 0,64 ± 0,03 (12) | < 0,05 |

## Patentansprüche

1. Bindungspartner für einen 5-HT5-Rezeptor zur Verwendung bei der Behandlung einer neuropsychiatrischen Störung.

2. Bindungspartner zur Verwendung nach Anspruch 1, wobei die neuropsychiatrische Störung Schizophrenie ist.

3. Bindungspartner zur Verwendung nach Anspruch 1, wobei die neuropsychiatrische Störung eine Psychose ist.

4. Bindungspartner zur Verwendung nach einem der Ansprüche 1 bis 3, wobei der Kᵢ-Wert für die Bindung des Bindungspartners an den 5-HT5-Rezeptor weniger als 10⁻⁶ M, vorzugsweise weniger als 10⁻⁷ M und insbesondere weniger als 10⁻⁸ M beträgt.

5. Bindungspartner zur Verwendung nach einem der Ansprüche 1 bis 4, wobei die Bindungsaffinität des Bindungspartners für einen 5-HT1A-Rezeptor höchstens um den Faktor 10 größer ist als für den 5-HT5-Rezeptor.

6. Bindungspartner zur Verwendung nach einem der Ansprüche 1 bis 5, wobei die Bindungsaffinität des Bindungspartners für den 5-HT5-Rezeptor größer ist als für den 5-HT1A-Rezeptor.
